# EUROPEAN PATENT APPLICATION

(11) **EP 0 539 092 A1**
(43) Date of publication of application: **28.04.1993**
(21) Application number: 92309391.8
(22) Date of filing: 15.10.1992
(51) Int. Cl.: C07C 269/00, C07C 271/12

(54) **Process for preparation of halopropargyl carbamates**

(30) Priority: 24.10.1991 US 782039
(71) Applicant: ROHM AND HAAS COMPANY, Philadelphia Pennsylvania 19105 (US)
(72) Inventor: Hsu, Adam Chi-Tung, Landsdale, Pennsylvania 19446 (US); Chang, Sou-Jen, Horsham, Pennsylvania 19044 (US)
(74) Representative: Smith, Julian Philip Howard

(57) **Abstract**

A process for preparing halopropargyl carbamates which avoids using phosgene as a starting material. N-butyl iodopropargyl carbamate is an especially preferred product of the novel process.

## Description

This invention concerns chemical processes for the preparation of halopropargyl carbamates.

Polyphase® fungicide is currently one of the leading paint mildewcides in the marketplace. The active ingredient is N-butyl iodopropargyl carbamate.

Troy Chemical Corporation in U. S.-A-3,923,870 discloses what is presumed to be the commercial process for preparing N-butyl iodopropargyl carbamate. ICI, in EP-A-14032, published June 6, 1990, disclose reacting an alkynol with an isocyanate and then iodinating. The Troy and ICI processes use butyl isocyanate which is believed to be produced from the reaction of butyl amine with phosgene. Isocyanates are generally toxic and are hazardous materials to handle. Troy's more recent process patent application, GB-A-2220000, published june 23 1989, discloses a method which uses as a starting material alkynyl chloroformate which is presumably produced from the reaction from propargyl alcohol and phosgene. Alkynyl chloroformate is also toxic, unstable, and hazardous. It is probably produced from phosgene. Although phosgene is a cheap raw material, it is also a very hazardous material. It is likely that the cost of production of N-butyl iodopropargyl carbamate is greatly influenced by the safe handling of phosgene.

According to Prashad, et al., Selective Monocarbamoylation of Symmetrical Diols with Alkyl Halides and Potassium Cyanate Using Phase-Transfer Catalysis, Synthesis Papers, 1989, 477, isocyanate can be produced *in situ*, by reacting potassium cyanate and alkyl halide in the presence of phase transfer agent, and directly reacts with an alcohol, preferably a diol, to produce the carbamate. Propargyl alcohol was not studied in this paper.

It is an object of the present invention to provide an improved process for preparation of halopropargyl carbamates. A further object is to provide a process ion which phosgene or butyl isocyanate need not be handled, and other hazardous raw materials need not be used. Production of N-butyl iodopropargyl carbamate is also an object of a preferred embodiment of the invention.

Accordingly in one aspect the present invention provides a process for preparing halopropargyl carbamate compounds, comprising reacting
(A) an alcohol of the formula 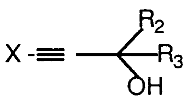 wherein R₂ and R₃ are each independently H or (C₁-C₄) alkyl, and X is H, Br, or I; with
(B) an alkyl halide of the formula

   R₁ X₁

   wherein R₁ is (C₁-C₈) alkyl, (C₃-C₈) alkenyl, (C₃-C₈) alkynyl, or benzyl, and X₁ is Cl, Br, I, or OSO₂R₄ wherein R₄ is (C₁-C₄) alkyl or phenyl optionally substituted with one or more of methyl, Cl, or N0₂; and
(C) an alkali metal cyanate of the formula MOCN, where M is K, Na or Li; in the presence of a catalyst.

The process of the invention is preferably run in an inert solvent such as such as nitrile, ester, ketone, DMF, and the like. Suitable catalysts are any which promote the desired reaction, especially phase transfer catalysts. Preferred catalysts are one or more selected from the group consisting of tetraalkylammonium halide, tetraalkyl ammonium sulfate, and crown ether. An especially preferred catalyst is tetrabutyl ammonium bromide.

Preferred alcohols are one or more selected from propargyl alcohol, 1,1-dimethyl propargyl alcohol, or iodopropargyl alcohol which is especially preferred. Contrary to the suggestion in the Prashad paper, supra, these mono-alcohols give a good yield.

A preferred process includes forming a carbamate intermediate of the formula 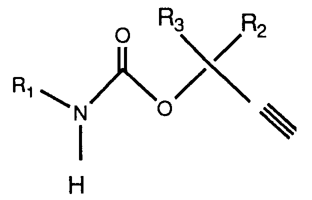
and reacting said carbamate intermediate with a halogen or halogen releaser in the presence of base. In such case, the initial alcohol is a propargyl alcohol rather than a halopropargyl alcohol. Suitable bases include NaOH, KOH, Na₂CO₃, and K₂C0₃ and the like. Suitable halogen or halogen releasers are Cl₂, I₂, Br₂, and 1,3-dibromo-5,5-dimethyl hydantoin and the like; I₂ is preferred.

Starting from the halopropargyl alcohol, the reaction can proceed in one step without the formation of said intermediate- In the single step version of the process of the invention, the preferred alcohol is iodopropargyl alcohol, the preferred alkyl halide is butyl halide, and the preferred halopropargyl carbamate compound is n-butyl iodopropargyl carbamate, with the resulting product therefore being N-butyl iodopropargyl carbamate.

Preferred alkyl halides include n-butyl bromide, n-butyl chloride, iodide, and sulfonates. Other suitable alkyl halides are methyl iodide, ethyl iodide, n-propyl chloride, n-propyl bromide, benzyl chloride, benzyl bromide, benzyl iodide, n-pentyl chloride, n-hexyl chloride, propargyl bromide, propargyl iodide, allyl chloride, allyl bromide, and allyl iodide- Reaction temperatures of below room temperature to the boiling point of the lowest boiling component of the reaction mixture are suitable; for the two step process the preferred reaction temperature is about 25°C-100°C for the first step, and about 0°C to 30°C for the second step. For the one step process, about 25°C-100°C is the preferred reaction temperature. Preferably the reaction is conducted in the presence of inert solvent, with nitrile, ester, ketone, and DMF being preferred.

The following examples illustrate a few embodiments of the invention; however, the invention should not be construed as being limited to these few illustrative embodiments.

### EXAMPLE I

### Synthesis of N-Butyl Propargyl Carbamate:

A mixture of n-butyl bromide (8.8 g, 0.064 mole), propargyl alcohol (3 g, 0.054 mole), potassium cyanide (6.5 g, 0.08 mole) and tetrabutylammonium bromide (1.7 g, 0.005 mole) in acetonitrile (45 mL) was heated at 70°C. under nitrogen for 22 hours. The insoluble precipitate was filtered off and the filtrate was concentrated on a rotary evaporator to give a yellow oil (6.7 g). Column chromatography (silica gel, 15/85 of ethyl acetate/hexane) gave a colourless oil (4.4 g, 53 %) of N-butyl propargyl carbamate. NMR (CDC₁₃, ppm): 4.75 (2H, s), 3.2 (2H, m), 2.5 (1H, s), 1.4 (4H, m), 1.0 (3H, t).; GC/MS (m/e): 155 (M+), 112 (100 %), 74, 57, 56.

### EXAMPLE II

### Iodination of N-Butyl Proparpyl Carbamate:

Iodine (3.3 g, 0.013 mole) was added in portions to stirred solution of the N-butyl propargyl carbamate (4.0 g, 0.026 mole) in ethanol (25 mL), water (10 mL) and 50 % sodium hydroxide (2.1 g, 0.026 mole) at 0-5°C. At the end of the iodine addition, the mixture was stirred at the same temperature for another 5 min. A commercial bleach (18.3 g, 5.25 %, 0.013 mole) was then added dropwise to the above solution keeping the temperature at 0-5°C. At the end of the bleach addition, the light yellow solution was stirred at the same temperature for one hour. Extraction with methylene chloride (2 x 70 ml) and evaporation of the solvent on a rotary evaporator gave crystalline residue (6.0g, 82.7%). Crystallization from hexane/ toluene gave the N-butyl iodopropargyl carbamate as needles; mp 60-62°C. NMR (CDCl₃, ppm): 4.85 (2H, s), 4.8 (1H, s), 3.2 (2H, t), 1.5 (2H, m), 1.4 (2H, m), 1.0 (3H, t).

### EXAMPLE III

### Preparation of N-Butyl Propargyl Carbamate:

N-butyl bromide (8.8 g, 0.064 mole) and propargyl alcohol (3.0 g, 0.054 mole) were added to a stirred suspension of KOCN (6.5 g, 0.080 mole) and tetrabutylammonium hydrogensulphate (1.8 g, 0.005 mole) in DMF (50 mL). A few drops of dibutyltin dilaurate were added. The mixture was stirred at room temperature for 40 hours and then heated to 70°C for 7 hours under nitrogen. After filtration, the filtrate was rotavaped to remove DMF. The oily residue (6.1 g) was then chromatographed (silica gel, 25/75 ethyl acetate/hexane) to give the carbamate (2.8 g, 33.8 %) as a colourless oil.

### EXAMPLE IV

### Preparation of N-Butyl Propargyl Carbamate:

A mixture of N-butyl bromide (7.3 g, 0.053 mole), propargyl alcohol (3.6 g, 0.064 mole), KOCN (4.3 g, 0.053 mole), tetrabutylammonium bromide (1.7 g, 0.005 mole) and dibutytin dilaurate (5 drops) in acetonitrile (50 mL) was heated at 70°C under nitrogen for 22 hr. The solids were filtered off and the filtrate was dried on the rotary evaporator to give a yellow oil (6.0 g). Column chromatography (silica gel, 30/70 ethyl acetate/hexane) gave the carbamate as a colourless oil (3.5 g, 42.4 % yield)

While the present invention has been described in considerable detail, various modifications and alternatives should become readily apparent to those skilled in the art.

## Claims

1. Process for preparing halopropargyl carbamate compounds, comprising reacting
(A) an alcohol of the formula 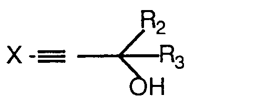 wherein R₂ and R₃ are each independently H or (C₁-C₄) alkyl, and X is H, Br, or I; with
(B) an alkyl halide of the formula
R₁ X₁
wherein R₁ is (C₁-C₈) alkyl, (C₃-C₈) alkenyl, (C₃-C₈) alkynyl, or benzyl, and X₁ is Cl, Br, I, or OSO₂R₄ wherein R₄ is (C₁-C₄) alkyl or phenyl optionally substituted with one or more of methyl, Cl, or N0₂; and
(C) an alkali metal cyanate of the formula MOCN, where M is K, Na or Li; in the presence of a catalyst.

2. Process according to claim 1 wherein said catalyst is tetraalkylammonium halide, tetraalkyl ammonium sulfate or crown ether, preferably tetrabutyl ammonium bromide.

3. Process according to claim 1 or 2 wherein said alcohol (A) is propargyl alcohol, iodopropargyl alcohol or 1,1-dimethyl propargyl alcohol.

4. Process according to any preceding claim wherein said alkyl halide is n-butyl bromide, n-butyl chloride, n-butyl iodide or an n-butyl sulfonate.

5. Process according to any preceding claim wherein said alcohol is iodopropargyl alcohol, said alkyl halide is butyl halide, and said halopropargyl carbamate compound is n-butyl iodopropargyl carbamate.

6. Process according to any preceding claim wherein X is I or Br and said halopropargyl carbamate compound is prepared in a single step.

7. Process according to any one of claims 1 to 4 wherein X is hydrogen, and the process comprises the steps of: (i) forming a carbamate intermediate of the formula 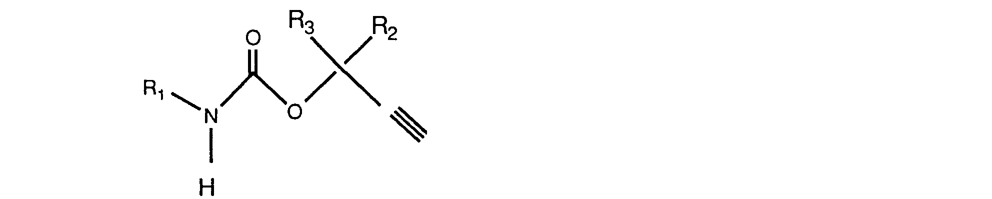 and (ii) halogenating said carbamate intermediate to form said halopropargyl carbamate product.

8. Process according to claim 7 wherein said halogenation comprises reacting said carbamate intermediate with a halogen or halogen releaser in the presence of a base, which base is preferably NaOH, KOH, Na₂CO₃ or K₂C0₃, and which halogen or halogen releaser is preferably Cl₂, I₂, Br₂ or 1,3-dibromo-5,5-dimethyl hydantoin.

9. Process according to claim 7 or 8 wherein said carbamate intermediate is n-butyl propargyl carbamate, said halogen-releaser is I₂, and the final product is n-butyl iodopropargyl carbamate.

10. Process according to any preceding claim wherein said reaction is conducted in the presence of an inert solvent, which solvent is preferably nitrile, ester, ketone, or DMF.

11. Process according to any preceding claim wherein the reaction is carried out at a temperature of between 25°C and 100°C, and in the case of a two-step reaction the second step is between 0°C and 30°C.

12. A halopropargyl carbamate compound obtainable by a process according to any preceding claim.
